# FASCICULE DE BREVET EUROPEEN

(11) **EP 2 713 869 B1**
(45) Date de publication et mention de la délivrance du brevet: **04.10.2017**
(21) Numéro de dépôt: 12727623.6
(22) Date de dépôt: 28.05.2012
(51) Int. Cl.: A61B 5/08, G01N 33/497

(54) **APPAREIL D'ANALYSE DIAGNOSTIQUE D'OXYDE NITRIQUE**
VORRICHTUNG ZUR DIAGNOSTISCHEN ANALYSE VON STICKOXID
APPARATUS FOR DIAGNOSTIC ANALYSIS OF NITRIC OXIDE

(30) Priorité: 26.05.2011 BE 201100326
(43) Date de publication de la demande: 09.04.2014
(73) Titulaire: Medi-Soft S.A., 1000 Bruxelles (BE)
(72) Inventeur: MARTINOT, Guy, Hastière 5540 (BE)
(74) Mandataire: Colens, Alain M.G.M.
(86) Numéro de dépôt international: PCT/EP2012/059932
(87) Numéro de publication internationale: WO 2012/160214

(56) Documents cités:
- EP-A2- 1 112 716
- EP-A2- 1 661 514
- WO-A1-2009/030957
- US-A1- 2010 081 955
- US-A1- 2011 077 545

## Description

L'invention concerne un appareil d'analyse diagnostique de l'air exhalé, notamment pour la détermination de la teneur d'un gaz endogène tel l'oxyde nitrique (NO, monoxyde d'azote) dans l'air exhalé, plus particulièrement l'air exhalé par la bouche d'un patient.

### Arrière-plan technologique de l'invention

On sait depuis de nombreuses années que l'oxyde nitrique endogène contenu dans l'air expiré est un marqueur de l'état d'inflammation des voies respiratoires d'un patient (voir p.e. WO93/05709 et WO95/02181, US2010/081955). On a toutefois constaté que pour une mesure fiable de la concentration en NO produite de façon endogène dans les voies respiratoires inférieures, notamment les poumons, il y avait lieu de faire inhaler et exhaler le patient par la bouche afin d'éviter de contaminer la mesure par du NO produit dans les voies respiratoires supérieures, notamment dans la cavité nasale.

La publication, Increased Nitric Oxide in Exhaled Air Of Asthmatic Patients, S.A. Kharitonov et al. The Lancet, Vol 343, January 15, 1994, divulgue que des patients asthmatiques ont des taux de NO plus élevés dans l'air expiré, en comparaison avec des sujets qui ne présentent pas cette maladie. Par ailleurs, ces teneurs en NO diminuent sensiblement après mise en place d'une thérapie adaptée. La détermination de la concentration en NO dans l'air exhalé peut donc permettre de diagnostiquer la maladie et de suivre la réponse du patient au traitement mis en place.

Diverses études ont été publiées avec des appareils de mesure de référence qui permettent de déceler avec précision des teneurs en NO de l'ordre du ppb (partie par millard) dans l'air exhalé notamment par la bouche (voir « Recommendations for Standardized Procedures for the Online and Offline Measurement of Exhaled Lower Respiratory NitricOxide and Nasal Nitric Oxide in Adults and Children - 1999 » publié dans Am J Respir Crit Care Med, Vol 160. Pages 2104 - 2117, 1999). Alors que les premiers appareils de mesure de NO dans l'haleine, notamment des appareils de laboratoire, faisaient essentiellement appel à des détecteurs basés sur le principe de la chimioluminescence (voir Endogenous Nitric Oxide in Single Exhalations and the Change during Exercise, M.G. Persson et al., Am Rev Respir Dis, Vol 148. Pages 1210-1214, publié en novembre 1993), on a ultérieurement cherché à simplifier la technologie et à réduire les coûts. Pour ce faire, les sondes électrochimiques s'imposaient à l'évidence. Dans ce but leur sensibilité a été améliorée, notamment en amplifiant et en traitant électroniquement le signal émis par ces sondes électrochimiques. Toutefois, les sondes électrochimiques, exigent un temps de réponse plus long avant de fournir un signal fiable (plateau stable) et ce temps de réponse est en général supérieur au temps d'expiration de la portion concernée du volume expiré par le patient. Ce temps d'expiration et/ou débit d'expiration est par ailleurs avantageusement contrôlé afin de garantir un échange suffisant entre les alvéoles pulmonaires et/ou les voies respiratoires inférieures et l'air en contact. Pour être fiable et comparable, la mesure sera standardisée.

Selon le document de brevet EP-1439781, alors qu'un détecteur basé sur les principes de la chimioluminescence permet une détermination quasi instantanée de la concentration en NO dans un flux gazeux, comme déjà mentionné un détecteur électrochimique requiert un temps de réponse plus long avant que le signal ne soit stabilisé. Les inventeurs visent à fournir une interface entre les paramètres dictés par des facteurs physiologiques (par exemple humidité, température etc.), et des paramètres dictés par des procédures médicales ou diagnostiques standardisées (débit de l'échantillon, durée) d'une part et des paramètres liés au détecteur d'autre part. Par ailleurs l'avantage à cet égard d'une mesure off-line faisant appel à un sac ou un ballon respiratoire, pour fournir une interface entre lesdits paramètres liés d'une part à la physiologie respiratoire et d'autre part aux propriétés du détecteur était déjà connu.

Selon le document de brevet EP-1439781 il est proposé un appareil de mesure de NO comprenant un capteur électrochimique destiné à déterminer la concentration en NO dans l'air exhalé et une admission par laquelle un sujet exhale à une pression et un débit prédéterminés, appareil de mesure comportant par ailleurs une chambre tampon avec un volume déterminé servant à accumuler temporairement une partie au moins de l'air exhalé et un dispositif de transfert de l'air exhalé au capteur, qui contrôle le passage de cet air exhalé sur le capteur à un débit régulier d'environ 0,5 à 15 cm3/s pendant une période supérieure à la durée de l'exhalation. Une fois passé sur le détecteur, l'air est expulsé vers l'extérieur de l'appareil, dans l'environnement.

### Buts de l'invention

La présente invention vise à fournir un moyen alternatif pour la détermination de la concentration d'un gaz, par exemple le NO, dans l'air exhalé d'un sujet moyennant un appareil de mesure comportant un détecteur à réponse lente, par exemple une sonde électrochimique.

De manière plus spécifique, le but de l'invention est de pouvoir analyser un volume limité de gaz, généralement inférieur à 700 ml, mais pouvant être aussi faible que 50 ml, avec un analyseur à réponse lente (par exemple supérieur à 20 voire 35 secondes) dont le débit doit rester important, par exemple supérieur à 3ml/sec, de préfrence supérieur à 15 ml/sec pour de nombreuses applications.

L'appareil selon l'invention doit bien entendu satisfaire les exigences de précision, sachant qu'il s'agit de mesurer des concentrations de NO dans l'air de l'ordre du ppb, tout en étant à faible coût.

### Résumé de l'invention

La présente invention propose de faire passer l'échantillon d'air exhalé accumulé dans un sac ou ballon collecteur, ou dans une chambre tampon, de façon contrôlée dans le détecteur électrochimique à un débit de 0,5 à 50 cm3/s de manière continue en le faisant recirculer dans ledit sac, ballon ou chambre tampon, et de prolonger cette opération jusqu'à stabilisation du signal mesuré par le détecteur électrochimique. Le gaz étant recyclé, le volume à analyser peut dès lors être très faible. Le temps de recyclage peut être très long (plusieurs minutes) car la teneur en composant gazeux à analyser reste pratiquement constante même après plusieurs passages, la consommation de ce composant durant l'analyse étant négligeable.

L'invention concerne donc un appareil de mesure d'un composant de l'haleine, en particulier de NO, dans l'air exhalé d'un sujet, comprenant une admission par laquelle l'appareil reçoit l'air exhalé par un sujet, un sac, un ballon ou une chambre tampon servant à accumuler temporairement une partie au moins de l'air exhalé, un capteur à réponse lente, par exemple un capteur électrochimique, destiné à déterminer la concentration en ledit composant, par exemple le NO, dans l'air exhalé, un dispositif de transfert de l'air exhalé de la chambre, du sac ou du ballon tampon au capteur, qui contrôle le passage de cet air exhalé sur le capteur à un débit régulier, par exemple d'environ 0,5 à 50 cm3/s, et une conduite qui fait retourner cet air dans le sac, le ballon ou la chambre tampon créant ainsi un circuit fermé.

Avantageusement, le circuit "sac ou ballon ou chambre tampon" - sonde - retour chambre tampon comporte une mise à l'air libre afin de pouvoir vider le sac, le ballon ou la chambre tampon pour un nouvel échantillonnage ou un étalonnage. Par ailleurs, la procédure d'analyse comprend avantageusement un temps initial de quelques secondes d'analyse en circuit ouvert afin d'éliminer l'effet de l'espace mort, le volume de gaz rejeté dans l'air ambiant étant largement supérieur au volume des conduites du circuit fermé.

Le dispositif de transfert de l'air exhalé de la chambre tampon au capteur contrôle avantageusement le passage de cet air exhalé sur le capteur à un débit régulier d'environ 0,5 à 50 cm3/s, de préférence supérieur à 15 cm3/s, et consiste avantageusement en une pompe, de préférence en parallèle avec une vanne de régulation, par exemple vanne à pointeau ("needle valve"), créant une boucle de régulation.

Cette disposition permet une plus grande flexibilité au niveau des débits d'échantillons d'air sur le détecteur, tout en garantissant une précision acceptable.

Alors que l'appareil selon l'état de la technique décrit dans EP-1439781 exige un contrôle de débit d'air expiré assez réduit sur le détecteur, à savoir de l'ordre de 10 ml/s, l'appareil selon l'invention permet de travailler à des débits supérieurs. L'un des avantages consiste en ce que le débit d'air expiré sur le détecteur peut être régulé avec une plus grande précision réduisant ainsi les imprécisions de l'appareil dues aux variations de débit d'air sur le détecteur.

L'invention permet donc de travailler avec une précision améliorée ou de construire un appareil utilisant des composants moins onéreux tout en maintenant une précision acceptable.

Un autre avantage de l'invention consiste en ce que l'on peut faire fonctionner l'appareil avec des volumes d'échantillon réduits, étant donné que le volume stocké temporairement est recirculé sur la sonde de mesure jusqu'à ce que celle-ci fournisse un signal stable. L'appareil selon l'invention peut contenir ou ne pas contenir d'autres dispositifs tels des détecteurs pour d'autres gaz que le NO, des filtres supplémentaires, des dispositifs spirométriques, ou des convertisseurs de gaz endogènes vers d'autres gaz aptes à être analysés plus facilement.

### Brève description du dessin

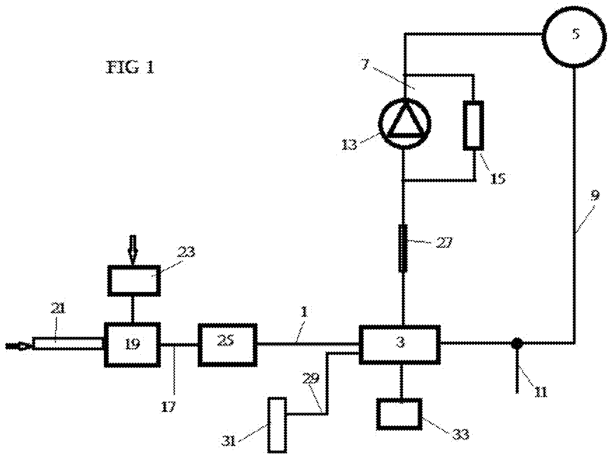

L'invention selon la revendication 1, est décrite ci-dessous en référence à la Figure 1 qui représente le dispositif de l'invention schématiquement et de façon assez simplifiée afin d'en faciliter la compréhension. Il est bien entendu que l'homme du métier fera appel à ses connaissances pour compléter les circuits par des sondes, moyens de régulation, boucles de contrôle, conduites etc. afin d'en améliorer les performances.

### Description détaillée de l'invention

Selon un mode de réalisation, l'appareil de la présente invention est destiné à mesurer la teneur en NO dans l'air exhalé d'un sujet. Cette mesure peut donner une indication sur l'état de santé des voies respiratoires du sujet. Dans la description qui suit et dans le cadre de l'invention, la chambre tampon peut être une chambre proprement dite à volume constant ou un ballon ou sac collecteur dont le volume est variable.

L'appareil comporte une admission 1 par laquelle l'appareil reçoit l'air exhalé par un sujet, à une pression et un débit prédéterminés, un sac tampon 3 servant à accumuler temporairement une partie au moins de l'air exhalé, un capteur électrochimique 5 destiné à déterminer la concentration en NO dans l'air exhalé, un dispositif de transfert de l'air exhalé 7 de la chambre tampon 3 au capteur 5, qui contrôle le passage de cet air exhalé sur le capteur à un débit régulier d'environ 0,5 à 50 cm3/s et retourne cet air dans la chambre tampon 3 via une conduite de liaison 9 en formant un circuit fermé.

L'appareil de l'invention comporte des électrovannes qui contrôlent les divers flux dans les conduites. Ainsi, l'air sortant du détecteur (5) est retourné dans le sac tampon au moins jusqu'à stabilisation du signal mesuré par le détecteur électrochimique 5.

La conduite 9 peut évidemment comporter un moyen, notamment une électrovanne, pour la mise à l'air libre 11 destiné entre autres à permettre une purge de l'espace mort en début de procédure d'analyse (circuit ouvert), ou de vider le sac tampon 3 et/ou à permettre le passage d'un flux de rinçage dans le circuit.

Une conduite 29 est avantageusement prévue pour relier la chambre tampon 3 à un réservoir 31 d'air ayant une concentration connue en gaz à analyser, et ce afin de permettre un étalonnage du détecteur à intervalles réguliers.

Le dispositif de transfert 7 de l'air exhalé du sac tampon 3 au capteur 5 contrôle le passage de cet air exhalé sur le détecteur 5 à un débit régulier d'environ 0,5 à 50 ml/s. Il consiste avantageusement en une pompe 13 de préférence doublée d'une vanne de régulation 15. Le débit est avantageusement contrôlé entre environ 10 et 25 ml/s, de préférence entre 15 et 20 ml/s.

L'admission 1 par laquelle l'appareil reçoit l'air exhalé par un sujet, à une pression et un débit prédéterminés, peut être connectée à la sortie 17 d'une vanne à trois voies 19. Ce type de vanne est connu dans l'état de la technique et a déjà été largement utilisé. A titre d'exemple, WO90/09572 décrit ce genre de vanne et son utilisation dans un appareil d'échantillonnage de l'air exhalé. En fait, le sujet aspire l'air, de préférence par la bouche, par une pièce buccale 21. La vanne ouvre un clapet d'admission de l'air préalablement filtré en 23. Le filtre 23 retient avantageusement le gaz à analyser qui serait également contenu dans l'air inspiré (gaz exogène). Lors de cette opération, la sortie 17 est obturée. Le sujet expire ensuite au travers de cette même pièce buccale, obturant ainsi le clapet d'admission d'air inspiré et ouvrant la sortie 17.

La pièce buccale peut évidemment être équipée d'un filtre bactériologique. Tant le patient que l'appareil sont ainsi protégés d'une éventuelle contamination bactérienne.

L'appareil de l'invention peut encore avantageusement comporter des moyens de contrôle de pression, de mesure de l'humidité de l'air à divers endroits dans le circuit, éventuellement connectés à des moyens de compensation adéquats.

Ainsi, le débit d'air au travers de la conduite 17 peut être mesuré et/ou régulé par un moyen adéquat 25 avant d'entrer dans le sac collecteur ou tampon 3. Il peut s'agir de manière connue d'un régulateur actif ou passif, tel un simple réducteur de débit et/ou un moyen indiquant au sujet qu'il doit expirer plus ou moins fort. Le débit d'exhalation est essentiellement standardisé dans un but diagnostique et est choisi afin d'assurer un contact suffisant entre l'air inspiré et les voies respiratoires. On considère généralement des débits expiratoires de l'ordre de 10 à 900 ml/s, de préférence de l'ordre de 50 ml/s. Il a été constaté que ce dernier débit permet une discrimination optimale entre une concentration normale en NO et une concentration révélatrice d'un état pathologique (asthme).

La chambre tampon peut présenter un volume de +/- 1000 cm3 mais pouvant accueillir des volumes d'échantillonnage de 200 à 700 cm3. Elle peut être équipée d'une mesure et/ou d'un contrôle de pression 33 localisée à l'intérieur de cette chambre. Comme la chambre tampon 3 consiste généralement en fait en un sac gonflable, il est avantageux de prévoir des moyens de sécurité permettant la mise à l'air libre de l'air exhalé en cas de surpression qui risquerait d'endommager la chambre tampon 3. De même une humidité trop importante, par exemple suite à la condensation de l'air exhalé dans la chambre tampon 3 doit être évitée afin de ne pas fausser les mesures sur le détecteur.

Il est bien entendu que le dispositif de transfert 7 doit être commandé lorsque la chambre tampon est suffisamment remplie d'air exhalé ou quand il est perçu que la chambre tampon contient l'échantillon voulu de l'air exhalé. En effet, il peut être intéressant de ne mesurer la teneur en NO qu'après une première phase d'exhalation plus ou moins longue qui est mise à l'air libre.

Afin de contrôler l'humidité de l'air exhalé envoyé sur le détecteur 5, on peut par exemple prévoir un absorbeur d'humidité ou un conduit en Nafion 27 qui, de manière amplement connue, équilibre l'humidité de l'air contenu dans ledit conduit et l'air ambiant.

On peut également prévoir des moyens de compensation de température des divers composants traversés par l'air exhalé afin d'essentiellement empêcher la condensation et/ou de stabiliser cette température.

Il est bien entendu que les divers composants de l'appareil de l'invention seront avantageusement aisément choisi par l'homme de l'art parmi les composants disponibles dans le commerce et adaptés à ce genre d'application.

Ainsi le détecteur peut être un détecteur commercialement disponible ou un tel détecteur qui a été adapté pour atteindre la sensibilité désirée, par exemple en faisant appel à un circuit électronique permettant de s'affranchir du bruit créé par une très haute amplification.

Au vu de ce qui précède, et vu les usages en la matière, une commande électronique de l'ensemble par programmation ou autre s'impose à l'évidence. Aussi, est-il avantageux de prévoir une mémoire enregistrant les données de plusieurs exhalations successives ou de divers patients etc. Ces aspects ne font toutefois pas partie de l'invention et ne seront pas détaillés ici.

Le dispositif de l'invention permet une construction compacte avec un espace tampon de volume assez réduit, à moins de 500 ml ou même moins de 100 ml tout en permettant l'analyse avec une précision suffisante de l'air exhalé contenu dans cette chambre tampon par un détecteur dont le temps de réponse est trop long que pour obtenir une réponse en temps réel, par exemple un détecteur électrochimique.

En vue de permettre la calibration du capteur, l'appareil de l'invention peut encore comporter une conduite 29 d'un réservoir 31 vers ladite chambre tampon 3 pour y amener un air de concentration connue en gaz à analyser.

Il est bien entendu que l'appareil décrit ci-dessus pour une application à la mesure d'oxyde nitrique (NO) dans l'air exhalé convient également à la mesure d'autres gaz dans l'air exhalé moyennant adaptation correspondante du détecteur, à savoir choix d'un détecteur approprié pour le gaz en question. L'homme du métier n'éprouvera aucune difficulté à sélectionner le composant adéquat.

L'invention propose donc de manière générale un appareil de mesure d'un composant dans l'air exhalé d'un sujet, comprenant une admission par laquelle l'appareil reçoit l'air exhalé par ledit sujet, une chambre tampon (ou sac ou ballon collecteur) servant à collecter et accumuler temporairement une partie au moins de l'air exhalé, un capteur, éventuellement à réponse lente, destiné à déterminer la concentration en composant dans l'air exhalé, un dispositif de transfert de l'air exhalé de la chambre tampon au capteur, qui contrôle le passage de cet air exhalé sur le capteur à un débit d'environ 0,1 à 100, de préférence 0,5 à 50 cm3/s, dans lequel l'appareil comprend une conduite apte à faire recirculer l'air exhalé de la sortie du capteur vers ladite chambre tampon (ou sac ou ballon collecteur), créant ainsi un circuit fermé. Le débit de l'air exhalé au niveau du capteur est avantageusement de 15 à 40 cm3/s, de préférence environ 20 cm3/s.

Des électrovannes contrôlent les flux d'air exhalé dans lesdites conduites et sont aptes à conduire cet air vers l'air libre.

Selon une forme d'exécution avantageuse, l'appareil comporte encore un moyen 25 pour déterminer la pression et/ou le débit de l'air exhalé vers la chambre tampon (ou sac ou ballon collecteur) .

## Revendications

1. Appareil de mesure de NO (monoxide d'azote) dans l'air exhalé d'un sujet, comprenant une admission (1) par laquelle l'appareil reçoit l'air exhalé par un sujet, un sac, ballon ou une chambre tampon (3) servant à accumuler temporairement une partie au moins de l'air exhalé, un capteur électrochimique (5) destiné à déterminer la concentration en NO dans l'air exhalé, un dispositif de transfert de l'air exhalé (7) du sac, ballon ou de la chambre tampon (3) au capteur (5), qui contrôle le passage de cet air exhalé sur le capteur (5) à un débit d'environ 0,5 à 50 cm3/s, comprenant l'appareil comprenant une conduite (9) apte à faire recirculer l'air exhalé de la sortie du capteur (5) vers la chambre tampon (3), créant ainsi un circuit fermé et comprenant en outre des électrovannes contrôlant les flux d'air exhalé dans lesdites conduites et aptes à conduire (11) cet air vers l'air libre, lesdites électrovannes étant réglées pour faire recirculer l'air exhalé de la sortie du capteur (5) vers la chambre tampon (3) jusqu'à stabilisation de la réponse du capteur.

2. Appareil selon la revendication 1 dans lequel le dispositif de transfert (7) est une pompe, éventuellement associée à une vanne de régulation en parallèle, de préférence une vanne à pointeau.

3. Appareil selon l'une des revendications précédentes dans lequel le débit de l'air exhalé, au niveau du capteur, est régulier et de l'ordre de 15 à 50 cm3/s, de préférence environ 20 cm3/s.

4. Appareil selon l'une des revendications précédentes comportant un moyen (25) pour déterminer la pression et/ou le débit de l'air exhalé vers la chambre tampon.

5. Appareil selon l'une des revendications précédentes dans lequel le sac collecteur ou le ballon est d'un volume de 1000 cm3, mais pouvant accueillir des volumes d'échantillonnage de 200 à 700 cm3.

6. Appareil selon l'une des revendications précédentes comportant encore une conduite (29) d'un réservoir (31) vers ladite chambre tampon (3) pour amener un air de concentration connue en gaz à analyser dans un but de calibration du capteur (5).

7. Appareil selon l'une des revendications précédentes **caractérisé en ce que** l'admission (1) par laquelle il reçoit l'air exhalé par un sujet, à une pression et un débit prédéterminés, est connectée à la sortie (17) d'une vanne à trois voie (19).

8. Appareil selon la revendication 7 **caractérisé en ce qu'**une pièce buccale (21) est connectée à l'entrée de la vanne (19).

9. Appareil selon la revendication 7 ou 8 **caractérisé en ce que** l'autre admission de la vanne (19) est connectée à un filtre (23) pour éliminer le NO inhalé de l'air ambiant.

10. Appareil selon l'une des revendications précédentes **caractérisé en ce qu'**il comporte des moyens de contrôle de température, de pression, de mesure de l'humidité de l'air à divers endroits dans le circuit, éventuellement connectés à des moyens de compensation adéquats.

11. Appareil selon l'une des revendications précédentes **caractérisé en ce qu'**il comporte un moyen (33) de mesure et/ou de contrôle de pression dans le sac, le ballon ou la chambre tampon (3).

12. Appareil selon l'une des revendications précédentes **caractérisé en ce qu'**il comporte un moyen de contrôle (27) de l'humidité de l'air exhalé envoyé sur le détecteur (5).

13. Utilisation d'un appareil selon n'importe laquelle des revendications précédentes pour la détermination de la concentration du NO dans l'air expiré.

## Patentansprüche

1. Apparat zur Messung von in der ausgeatmeten Luft einer Person enthaltenem NO (Stickstoffmonoxid), umfassend einen Einlass (1), durch den der Apparat die von einer Person ausgeatmete Luft empfängt, einen Beutel, einen Ballon oder eine Pufferkammer (3), die dazu bestimmt sind, mindestens einen Teil der ausgeatmeten Luft zeitweise zu akkumulieren, einen elektrochemischen Sensor (5), der dazu bestimmt ist, die NO-Konzentration in der ausgeatmeten Luft zu bestimmen, eine Transferierungsvorrichtung zur Überführung der ausgeatmeten Luft (7) von dem Beutel, dem Ballon oder von der Pufferkammer (3) zu dem Sensor (5), die den Durchgang dieser ausgeatmeten Luft auf dem Sensor (5) bei einer Leistung von etwa 0,5 bis 50 cm³ / s kontrolliert, wobei der Apparat eine Leitung (9) umfasst, die dazu geeignet ist, die ausgeatmete Luft von dem Ausgang des Sensors (5) zu der Pufferkammer (3) unter Bildung eines geschlossenen Kreislaufes zurückzuführen, und ferner Elektroventile umfasst, die die Ströme der in die besagten Leitungen ausgeatmeten Luft kontrollieren und dazu geeignet sind, diese Luft ins Freie zu führen (11), wobei die besagten Ventile geregelt sind, um die ausgeatmete Luft von dem Ausgang des Sensors (5) zu der Pufferkammer (3) zurückzuführen bis zu einer Stabilisierung der Antwort des Sensors.

2. Apparat nach dem Anspruch 1, bei dem die Transferierungsvorrichtung (7) eine Pumpe ist, der eventuell ein Regelventil, vorzugsweise ein Nadelventil, parallel zugeordnet ist.

3. Apparat nach irgendeinem der vorangehenden Ansprüche, bei dem der Durchlauf von ausgeatmeter Luft auf der Ebene des Sensors regelmäßig ist und in der Größenordnung von 15 bis 50 cm³ / s, vorzugsweise von ca. 20 cm³ / s liegt.

4. Apparat nach irgendeinem der vorangehenden Ansprüche, umfassend ein Mittel (25) zur Bestimmung des Druckes und / oder des Durchlasses der zu der Pufferkammer hin ausgeatmeten Luft.

5. Apparat nach irgendeinem der vorangehenden Ansprüche, bei dem der Sammelbeutel oder der Ballon ein Volumen von 1000 cm³ aufweist, der jedoch in der Lage ist, Proben mit einem Volumen von 200 bis 700 cm³ aufzunehmen.

6. Apparat nach irgendeinem der vorangehenden Ansprüche, umfassend ferner eine Leitung (29) eines Behälters (31) zu der besagten Pufferkammer (3), für die Zufuhr einer zu analysierenden, eine bekannten Gaskonzentration aufweisenden Luft zwecks Kalibrierung des Sensors (5).

7. Apparat nach irgendeinem der vorangehenden Ansprüche,
**dadurch gekennzeichnet, dass** der Einlass (1), durch den er die von einer Person ausgeatmete Luft bei einem Druck und einer Leistung, die vorgegeben sind, empfängt, am Ausgang (17) eines Dreiwegeventils (19) geschaltet ist.

8. Apparat nach dem Anspruch 7,
**dadurch gekennzeichnet, dass** ein Mundstück (21) am Eingang des Ventils (19) angeschlossen ist.

9. Apparat nach Anspruch 7 oder 8,
**dadurch gekennzeichnet, dass** der andere Eingang des Ventils (19) mit einem Filter (23) verbunden ist, um aus der Umgebungsluft das ausgeatmete NO zu eliminieren.

10. Apparat nach irgendeinem der vorangehenden Ansprüche,
**dadurch gekennzeichnet, dass** er Mittel zur Kontrolle der Temperatur, des Druckes, zur Messung der Feuchtigkeit der Luft an verschiedenen Stellen innerhalb des Kreislaufes aufweist, die eventuell mit entsprechenden Kompensationsmitteln verbunden sind.

11. Apparat nach irgendeinem der vorangehenden Ansprüche,
**dadurch gekennzeichnet, dass** er ein Mittel (33) zur Messung und / oder zur Kontrolle des Druckes in dem Beutel, dem Ballon oder der Pufferkammer (3) umfasst.

12. Apparat nach irgendeinem der vorangehenden Ansprüche,
**dadurch gekennzeichnet, dass** er ein Mittel (27) zur Kontrolle der Feuchtigkeit in der zum Sensor (5) geführten ausgeatmeten Luft umfasst.

13. Verwendung eines Apparates nach irgendeinem der vorangehenden Ansprüche zur Bestimmung der NO-Konzentration in der ausgeatmeten Luft.

## Claims

1. Apparatus for measuring NO (nitrogen monoxide) in exhaled air of a patient, comprising - an inlet (1) by which exhaled air of the patient is supplied to the apparatus , - a bag, balloon or buffer chamber (3) for accumulating at least part of the exhaled air, - an electrochemical sensor (5) for determining the NO concentration in the exhaled air, - a device for transferring the exhaled air (7) from the bag, balloon or buffer chamber (3) to the sensor (5) at a flow rate of 0,5 to 50 cm3/s , the apparatus comprising a conduit (9) to recirculate exhaled air from the outlet of the sensor (5) to the buffer chamber (5), creating a closed circuit, and furthermore comprising electrovalves controlling the flux of exhaled air in such conduit and able to lead (11) this air to the ambiant air, said electrovalves being controlled in order to recirculate the exhaled air from the outlet of the sensor to the buffered chamber (3) until stabilisation of the response of the sensor.

2. Apparatus according to claim 1 wherein the transfer device is a pump, possibly associated with a regulating valve in parallel, preferable a needle valve.

3. Apparatus according to one of the preceding claims wherein the flow rate of exhaled air, at the level of the sensor, is regular and of the order of 15 to 30 cm3/s, preferably about 20 cm3/s.

4. Apparatus according to one of the preceding claims wherein the comprising a means (25) for determining the pressure and/or the flow of exhaled air towards the buffer chamber.

5. Apparatus according to one of the preceding claims wherein the collecting bag or the balloon has a capacity of 1000 cm3, but able to receive sample volumes of 200 to 700 cm3.

6. Apparatus according to one of the preceding claims comprising furthermore a pipe (29) from a reservoir (31) to said buffer chamber (3) for leading an air with known concentration of gas to be analysed for calibration purpose of the sensor (5).

7. Apparatus according to one of the preceding claims **characterized in that** the inlet (1) by which it is fed with exhaled air of a patient, at a determined pressure and flow rate, is connected to an outlet (7) of a three way valve (19).

8. Apparatus according to one of the preceding claims **characterized in that** a mouth piece (21) is connected to the inlet of the valve (19).

9. Apparatus according to the claim 7 or 8 **characterized in that** the other inlet of the valve (19) is connected to a filter (23) for eliminating the inhaled NO of the ambiant air.

10. Apparatus according to one of the preceding claims **characterized in that** it comprises control means for temperature, pressure, humidity measurements of the air at various location of the circuit, possibly connected to appropriate compensation means.

11. Apparatus according to one of the preceding claims **characterized in that** it comprises a means for measuring and/or controlling the pressure in the bag, the balloon or buffer chamber (3).

12. Apparatus according to one of the preceding claims **characterised in that** it comprises a control means (27) for the humidity of exhaled air sent to the sensor (5).

13. Use of an apparatus according to any of the preceding claims for the determination of the NO concentration in exhaled air.
